# EUROPEAN PATENT SPECIFICATION

(11) **EP 2 521 768 B1**
(45) Date of publication and mention of the grant of the patent: **09.08.2017**
(21) Application number: 11715051.6
(22) Date of filing: 04.01.2011
(51) Int. Cl.: C12M 1/107, C05F 3/00, C05F 17/02

(54) **APPARATUS AND PRECULTURE TANK FOR BIOMETHANATION OF BIOMASS**
VORRICHTUNG UND VORKULTURETANK ZUR BIOMETHANATION VON BIOMASSE
APPAREIL ET RÉSERVOIR DE PRÉCULTURE POUR LA BIOMÉTHANATION DE LA BIOMASSE

(30) Priority: 04.01.2010 IN 15MU2010
(43) Date of publication of application: 14.11.2012
(73) Proprietor: Kirloskar Integrated Technologies Private Limited, Kothrud Pune 411 038 (IN)
(72) Inventor: MATE, Nitant Vishnu, Pune 411 004 (IN); GOYAL, Devendra, Jayant, Pune 411 037 (IN); JOSHI, Ashwin, Sharad, Satara 415 523 (IN); KHOT, Nikhil, Appasaheb, Kolhapur 416 003 (IN); GANU, Shirish, Madhav, Pune 411 052 (IN)
(74) Representative: Rusby-Gale, Daniel Matthew
(86) International application number: PCT/IN2011/000002
(87) International publication number: WO 2011/080766

(56) References cited:
- EP-A1- 1 930 404
- WO-A1-92/13084
- WO-A2-2007/052306
- WO-A2-2009/103866
- US-A- 4 022 665
- US-A- 4 040 953
- US-A- 4 252 901
- DEMIRER ET AL: "Anaerobic biogasification of undiluted dairy manure in leaching bed reactors", 13 November 2007 (2007-11-13), WASTE MANAGEMENT, ELSEVIER, NEW YORK, NY, US, PAGE(S) 112 - 119, XP022342537, ISSN: 0956-053X the whole document

## Description

### FIELD OF INVENTION:

This invention relates to a method of biogas generation.

Particularly, the present invention relates to a dual anaerobic fermentation process for biogas generation using microbial consortia.

More particularly the present invention relates to a combined dual anaerobic fermentation process for biogas generation and in two or more different anaerobic digesters.

### GLOSSARY:

### Biomethanation:

Biomethanation is the formation of methane, a metabolic by product in anoxic conditions by microbes known as methanogens under anaerobic condition.

### Biomass:

Biomass is defined as the total amount of living material in a given habitat. Herein biomass is referred to as any carbonaceous organic substrate including, but not limited to, sewage sludge, forestry waste, food waste, agricultural waste, municipal waste, agricultural feeds, agricultural produce, and the like.

### Dual process of biomethanation:

Herein defined as a biomethanation process in solid phase / state as one step and biomethanation process in liquid phase / state as the other step

### Hydraulic retention time (HRT):

The hydraulic retention time (HRT) is a measure of the average length of time that a substance / material / compound remains in a constructed reactor.

### Methane digester:

Methane digesters are anaerobic (low or no oxygen) chambers which facilitate the breakdown of manure (substrate) by anaerobic bacteria with the release of methane and other gases as byproducts of their metabolism, including ammonia, nitrogen, hydrogen sulfide, and sulfur dioxide. Herein substrate used is any biomass as defined above.

### Liquid state methane digester:

Herein defined as a methane digester wherein the leachates from solid state methane digester, fresh culture and feed are in liquid state, i.e. in a flowable form.

### Solid state anaerobic fermentation:

A process of anaerobic fermentation wherein the contents of the digester are in a non pump-able i.e. dry form- it may have considerably high percentage of liquid absorbed in the solid mass.

### Solid state methane digester:

Herein defined as a methane digester wherein culture, feed is in a moist but solid state i.e. the contents of the digester are in non pump-able form.

### Total solids (T.S.):

The total content of suspended and dissolved solids in liquid

### BACKGROUND OF THE INVENTION:

Anaerobic digestion is a biological process to degrade organic matter to produce biogas which is a renewable energy source and a sludge that could be used as fertilizer. In the absence of oxygen (anaerobic digestion), the organic matter is degraded partially by the combined action of several types of micro-organisms. A succession of biological reactions takes place leading to the formation of biogas and sludge. The bacteria which carry out these reactions exist in natural state in the liquid manure and the anaerobic ecosystems; it is not necessary to add more, they grow and multiply naturally in a medium without oxygen.

Anaerobic digestion is a series of processes in which biodegradable material is broken down by microorganisms and biochemical processes in the absence of oxygen and is widely used to treat wastewater. Anaerobic digestion is also widely used as a renewable energy source because the process produces methane rich biogas suitable for use as a source of energy helping replace fossil fuels as also, the nutrient-rich digestate can be used as fertilizer. The digestion process begins with bacteria assisted hydrolysis of the biomass materials to break down insoluble organic polymers such as carbohydrates, proteins, lipids and the like into some variety of sugars and / or amino acids, and make them available for another consortium of bacteria. Acidogenic bacteria then convert the sugars and amino acids into carbon dioxide, hydrogen, ammonia, and organic acids. Acetogenic bacteria further convert these resulting organic acids primarily into acetic acid and partially into other volatile fatty acids, along with additional ammonia, hydrogen, and carbon dioxide. Methanogens, finally convert these products to methane and carbon dioxide. This conversion is brought about by various processes and using substrates in different forms, phases i.e. wet and dry. Dry phase fermentation solves the scum formation problems as like created in the wet phase process of biomethanation. The slurry developed after biomethanation process is let out or is dumped out, hereby creating lose of microbes which succeeding can be utilized for biogas generation and manure production, and creating minimum amounts of than produced slurry and / or sludge.

Utilizing anaerobic digestion technologies help to reduce the emission of greenhouse gasses in a number of ways:
Replacement of fossil fuels
Reducing methane emission from landfills
Displacing industrially-produced chemical fertilizers
Reducing electrical grid transportation losses; as the electricity produced by a biogas plant is invariably consumed by localized consumers.

Herein is an option developed to overcome the above mentioned limitations with regards to single phase digestion biomethanation process. Some of the current technologies available for anaerobic digestion and their shortcomings are as follows.

### PRIOR ART:

1) Patent Application (WO/2007/096392) discloses "BIOREACTOR FOR METHANIZATION OF BIOMASS HAVING A HIGH SOLIDS FRACTION."
   **Abstract:** A bioreactor having improved gas yield is specified, in which the necessary residence time of the biomass in the rotting vessel is decreased. On fermentation of dry, that is to say non-pumpable, biomass, owing to the moisture present in the biomass, percolating juices, what is termed percolate, are formed and are taken off via a drainage system and, if appropriate, is recirculated from the top onto the biomass to be fermented. It has now turned out that the biogas yield is significantly increased, in the region between 10% and 40%, when the resultant percolate is not taken off immediately via the drainage system, but is backed up in the rotting vessel up to a certain level. This is achieved in terms of the device in such a manner that the rotting vessel is designed so as to be liquid-tight, that is to say even the flap for charging and unloading the rotting vessel has to be made in a liquid-tight manner, and also must be constructed in a correspondingly stable manner in order to withstand the resultant liquid pressure. By means of the combination of the existing percolate drainage system with a percolate control unit it is possible to set the liquid level of the percolate in the biomass to be fermented and to control it in such a manner that the biogas production rate or the biogas yield is maximal.
   Limitation: The patent claims that the percolate from the first digester is stored in a rotting vessel which if desirable is recirculated from the top onto the biomass. No attempt has been made to convert the rotting vessel into another methane generating digester, as also the present entire process is anaerobic.
2) United States Patent Application No 7,144,507 discloses "DRY CYCLE ANAEROBIC DIGESTER".
   Abstract: The present invention provides a digester for handling waste or contaminated materials. A process and an apparatus for processing are disclosed. A Dry Cycle Anaerobic Digester (DCAD) uses tanks to perform aerobic and anaerobic digestion to eliminate the waste, while producing little or no sludge.
   Limitation: The invention claims handling waste in liquid form storing it for a defined period and thereby emptying the tanks followed by drying of the tank. This does not have any effect on digester size. As also the process is aerobic and anaerobic, whereas the present invention describes a dual state anaerobic process.
3) Patent Application No WO/2007/075762 discloses "ANAEROBIC PHASED SOLIDS DIGESTER FOR BIOGAS PRODUCTION FROM ORGANIC SOLID WASTES."
   Abstract: The present invention provides methods for the generation of methane by a two phase anaerobic phase system (APS) digestion of organic substrates. Also provided is a device for practicing the methods of the invention. The APS-digester system is a space-efficient, high-rate solids digestion system. The APS-digester system consists of one or more hydrolysis reactors, a buffer tank and one biogasification reactor.
   Limitations: This invention describes biogas production in two stages: hydrolysis and methanization. In the first hydrolytic reactor, volatile fatty acids are produced, which are converted into biogas in the second hydrolytic reactor. Thus the process needs two reactors wherein only the second reactor is for anaerobic biomethanation.
4) Patent Application No WO 2006/017991 discloses "Stepped Sequential Treatment method for municipal domestic refuse."
   Abstract: The present invention provides a treatment method of municipal domestic refuse. In the method the organic matter processes an anaerobic fermentation; the obtained methane can be helpful to burning to generate electricity. The biogas residue from the anaerobic fermentation can be used as a culture material for growing edible mushrooms. The residue discharged from edible mushrooms can be used to cultivate earthworm. Besides the organics, the other substance of the municipal domestic refuse will be incinerated to generate electricity. The present invention realizes a comprehensive utilization of waste resource.
   Limitations: The process is restricted only for municipal refuse. No treatment is specified for any other type of waste.
5) US 4 022 665 discloses a two phase anaerobic digestion process.
6) EP 1 930 404 discloses a method and device for the anaerobic fermentation of organic material.

### BRIEF DESCRIPTION OF DRAWINGS:

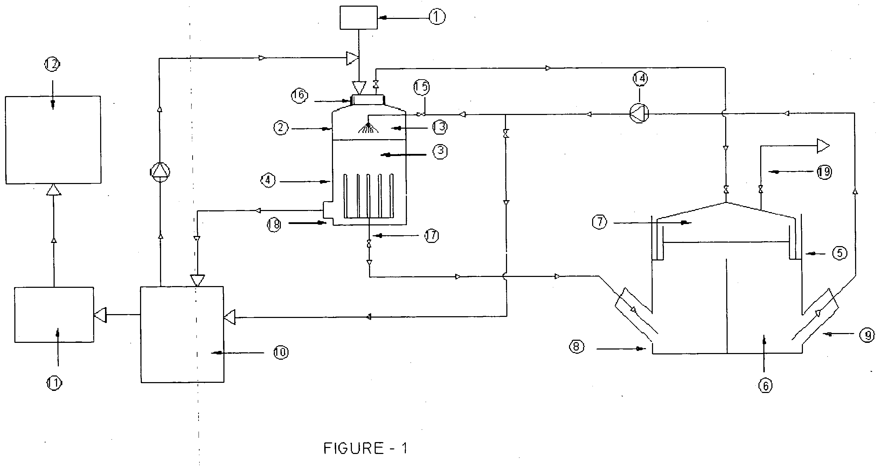

The present invention will be more fully understood and appreciated by reading the following detailed description in conjunction with the accompanying drawings. Figure 1 is the process flow diagram of dual phase digestion process using the solid state digester and the liquid state digester of the present invention in which;
Part list:
1) Feed Storage Tank
2) Solid State Methane Digester
3) Reaction chamber of solid state methane digester
4) A vertical perforated unit (tube)
5) Liquid State Methane Digester
6) Reaction chamber of liquid state methane digester
7) Biogas Storage Vessel (Dome)
8) Inlet port for collecting percolate
9) Outlet port for recycling the percolate
10) Culture Preparation Tank
11) Filtration Unit
12) Manure Preparation Unit
13) Spray recirculation system
14) Solid handling pump
15) Control valves/ Regulators
16) Insulated Feed Inlet port
17) Leachate outlet port connected to the liquid digester
18) Digested material outlet port
19) Gas outlet port

### DISCLOSURE OF THE INVENTION:

It is an object of the present invention to provide a method of biomethanation from biomass by combined solid and liquid state anaerobic fermentation.

An object of the present invention is to provide a method of biomethanation from biomass in two phases i.e. solid and liquid.

A further object of the present invention is to overcome problems associated with floating layers of scum formation which reduces the output of the biomethanation process.

A further object of the present invention is to reduce the digester size as compared to existing anaerobic digesters.

A further object of the present invention is to reduce the overall hydraulic retention time of the biomethanation process.

Still further object of the present invention is to provide a method for biomethanation of organic solid waste feeds, which utilizes minimum natural resources like water, electricity etc & can handle heterogeneous waste in the same digester scheme.

Another object of the present invention is to utilize the percolate produced through percolating units from the dry state digester biomass for further biogas production in other i.e. liquid state methane digester.

Yet another object of the present invention is to generate a self sustaining system, generation of biogas, generation of fuel, producing manure and producing minimum liquid effluent.

Yet another object of the present invention is to reduce waste water as compared to liquid state digestion systems and therefore reduce the requirement of equipment for managing the effluent stream.

Another object of the present invention is to provide a fast, economic and efficient biomethanation process.

Still another object of the present invention is to reduce the capital cost of biogas generation process.

Further object of the present invention is to reduce the processing and drying needs to use the non-digested biomass as manure or base material for organic fertilizer.

### SUMMARY OF THE INVENTION:

According to one aspect of the present invention, there is provided an apparatus as defined in claim 1 hereinafter.

According to another aspect of the present invention, there is provided a biomethanation process as defined in claim 9 hereinafter.

The present invention envisages a combined dual biomethanation process and in two or more different digesters. The number of digesters depends upon the retention time of the biomass used for the biomethanation process and the choice of the designer of the system. In solid state methane digester, the one phase, bacterial cultures developed for a specific feed material and the biomass is mixed in a desired ratio by mechanical means. The leachates generated in the solid state methane digester are collected at the base of the reaction chamber of the solid state methane digester by means of percolating pipes or other appropriate mechanism . The percolates produced are re-circulated by means of sprinkler or other appropriate arrangement incorporated in the solid state methane digester and in fluid communication with the liquid state methane digester. The contents of liquid state methane digester is heated and stirred occasionally as required, therein for producing methane rich gas, wherein, both the methanogenic digesters are maintained under anaerobic conditions. The process thus maintains the required temperature for microbial activity for biogas generation in both solid state and the liquid state methane digesters due to recirculation of lechates produced in the biomethanation process. The liquid state methane digester is fed with specific cultures to convert readily degradable organic matter like sugars and volatile fatty acids into biogas. Simultaneously, heating of liquid state methane digester and recirculation of the culture into the solid state methane digester helps improve the digestion rate. Part of the sludge produced from the solid state methane digester and some portion of the slurry produced from the liquid state methane digester is carried into a culture preparation unit for use as culture for next cycle of biogas production. The remaining solids and liquid is than filtered through appropriate filtration and / or drying units for converting it into manure of desired consistency. The biogas produced from both the digesters is collected in a common gas storage unit.

The present invention results into overall reduction in retention time for biomethanation. Both the digesters are heated between temperatures of about 30 Degree Centigrade to about 40 Degree Centigrade for mesophilic cultures. The temperature range is varied based on the type of bacteria used, viz. mesophilic, thermophilic, and the like. The methane rich gas generated is collected in a gas collecting assembly. The resultant produce i.e. methane and carbon dioxide (CO₂) containing biogas may be used for cooking purposes or for generating electricity or as vehicle fuel, etc., either as is or after cleaning and / or compressing to higher pressures. This mixture can also be converted to purified methane and compressed to replace CNG and used in vehicles or other applications. It could even be introduced in natural gas pipelines to add to their existing capacity.

The said process is thus a self sustaining system generating biogas from substrate, generation of fuel, producing manure, resulting in minimum effluent slurry.

### DETAILED DESCRIPTION OF THE PREFERRED EMBODIMENTS:

The foregoing objects of the said invention are accomplished and the problems and shortcomings associated with the prior art techniques are overcome by the present invention as described below in the preferred embodiment.

Embodiments of the invention are discussed below with reference to Figure 1. In a preferred embodiment, the process comprises two or more anaerobic reactors or digesters a culture preparation tank, a filtration unit, a manure preparation unit and a gas storage unit. The two stages / phases i.e. solid state methanogenesis and liquid state methanogenesis are carried out separately in the said two types of digesters. The number of digesters varies depending on the retention time of the biomass used. The first and the second or the one and the other phase are carried out in presence of microorganisms. The reactors are provided with facilities for temperature control and stirring mechanism as required.

In a preferred embodiment, the process of biomethanation comprises two or more methane digesters (2), (5), gas collecting unit (7), culture preparation tank (10), filtration unit (11) and manure preparation unit (12). The solid state methane digester (2) comprising a reaction chamber (3) for conversion of biomass into biogas, a vertical perforated unit probably tube (4), spray recirculation system (13), leachate outlet port (17) is in fluid communication with the liquid state methane digester (5), digested material outlet port (18) which is in communication with the culture preparation tank (10) and a fixed gas collecting chamber. The dry feed / biomass and substrate specific culture is introduced into the solid state methane digesting tank (2) from the feed storage tank (1). The feed / biomass preferably used are agro residues, oilcakes like paddy straw, wheat straw, maize, Napier grass, press mud, castor, sal, food waste, biodegradable municipal waste and alike. The total solids of the biomass preferably are in the range of 15-20 percent. The organic solid waste is digested by addition of specially developed microbial population capable of producing required enzymes. The microbial consortium is specifically prepared for a particular feed as a target and is enriched with natural microbial mixtures such as cow-dung, sewage and the like, by a process of restricting its nutrition to the subject feed over a period of time. Once enriched, this consortium can be propagated and made available for deploying in the reactor. The said solid state methane digester (2) has a percolation unit / tube (4) internally connected in parallel to the base of the reaction chamber of said solid state methane digester (3) to facilitate percolation of lechates from the feed present in the said digester (2). The process maintains the required temperature for microbial activity for biogas generation due to recirculation of lechates produced by the biomethanation process. The lechates are heated to a desired temperature for optimum performance of the bacterial consortium present in the digesters. The organic solid waste is digested to produce biogas, which generates percolate and solid digestate. The solid digestate is further utilized for feed and manure preparation. A sprinkler and/ or spray recirculation system (13) is introduced into the said solid state digester (2) which sprinkles digested slurry on top of the reaction mixture from the said liquid state methane digester (5) and collects percolate and / or lechates in a manifold created at the base of the solid state methane digester (2). The lechates produced from individual percolation unit are collected through a manifold into the said liquid state methane digester (5). The sprinkler (13) is in fluid communication with the said liquid state methane digester (5) and is suspended internally in the head space of the said solid state methane digester (2). The solid state methane digester (2) has a conduit /outlet arranged (18) at the lower region of the digesting tank for discharging sludge from the digesting tank, which is in communication with the culture preparation tank (10). The biomass is passed through the reaction chamber (3) for a period of time (about 5 to 20 days) sufficient for the feed mixture to be anaerobically digested. The period for degradation of biomass to biogas is variable, which depends on the retention time of the substrate used.

The liquid state methane digester (5) consists of a reaction chamber (6) and a flexible biogas collecting vessel preferably a dome shaped (7) for extraction of biogas generated from solid state methane digester (2) and liquid state methane digester (5). The biogas collecting vessel (7) / dome is mounted in the head region of the liquid state methane digester (5), which is movable and displaces gas by vertically upward movement. The liquid state methane digester (5) has provisions for collecting the percolate at the lower region of the digester (8) and an outlet at another lower end (9) for recycling the digested slurry into the solid state methane digester (2) by means of spray pumps and dispensers. The sludge produced is further filtered through a filtration unit (11) which may be sand filter or any other appropriate filter unit. The filtered sludge is finally dispatched for manure preparation into the manure preparation unit (12) manually. The number of solid state methane digesters varies with respect to the retention time of the biomass / substrate used for biogas generation. The spray recirculation system (13) maintains the desired temperature conditions inside the solid state methane digester (2) which in turn is controlled by solid handling pump (14). In the said liquid state methane digester (5), the conversion into biogas is brought by housing enriched microbial consortia, leachates and / or percolates from said solid state methane digester (2). The conversion into biogas is brought by addition of enriched microbial consortia, followed by heating it between temperature ranges of about 30 Degree Centigrade to about 40 Degree Centigrade or as may be required by the consortia of microbes, with or without occasional stirring. The reactor size is optimized taking into consideration the microbial population and retention time required for digestion. Introduction of the liquid state methane digester optimizes biogas generation from the non digested slurry which otherwise is disposed off.

Valves and regulators (15) or any other appropriate flow control mechanism are introduced to control the flow of lechates/ slurry. The culture preparation tank (10) is than regularly fed with the substrate and portion of the solid digested material from the solid state methane digester (2). The gas generated from the digesters is collected in storage vessel. The mixture produced in the said culture preparation tank (10) is utilized as feed further to obtain biogas.

The said non digested material prepared in the said culture preparation unit (10) is introduced partly in the said solid state methane reactor along with fresh biomass and partly treated for manure preparation. The said non digested material is introduced onto filtration tank (10) for draining excess water / liquid present in the solid digested material. The said non digested material further is subjected for composting to manure (12) in order to achieve the desired quality of carbon to nitrogen ratio. Biogas generated from both the digesters (2, 5) is collected in a gas collecting assembly which further can be utilized for cooking purposes or generating electricity or other productive uses like vehicle fuel with or without cleaning. Biogas can also be converted to purified methane and compressed to replace CNG in vehicle. The said process reduces the hydraulic retention time for the biomethanation process to 5 to 20 days depending on the substrate. The said process also reduces water consumption by about 50 percent as to that required by conventional method of biomethanation. The said process introduces dual biomethanation process by introduction of liquid state methane digester (5).

While considerable emphasis has been placed herein on the specific steps of the preferred process and components of the preferred embodiment, and many details have been set forth for purpose of illustration, it will be appreciated that many alterations can be made and that many modifications can be made in the preferred embodiment without departing from the principles of the invention. These and other changes in the preferred embodiment as well as other embodiments of the inventions will be apparent to those skilled in the art from the disclosure herein, whereby it is to be distinctly understood that the foregoing descriptive matter is to be interpreted merely as illustrative of the invention and not as a limitation.

The invention is further described with the help of following non limiting illustrations.

### Illustration 1:

### 1) Fresh Napier grass is used as substrate.

Fifteen kilograms of fresh Napier grass with total solids (TS) ranging from 20 to 25 percent, is pulverized upto 3 to 4 mm and allowed to undergo the said process of biomethanation.

The results observed are as follows:

| Sr. No. | Substrate | Digester to Gas Ratio | Biogas Potential recovery (Liters/kg T.S.) | Retention Time (Days) | Expected Biogas production (liters/day) | Actual Biogas production (liters/day) |
|---|---|---|---|---|---|---|
| 1 | Fresh Napier grass | 1.2-1.5 | 250 - 350 | 18 | 900 | 1300 - 1400 |

### Observations:

As the actual biogas output is about 50 percent more than the expected, the biogas to digester volume ratio is improved from 0.85 to about 1.2 - 1.5. The retention time is reduced to 18 days, whereby the conventional wet biogas digester producing biogas at similar gas potential recovery required over 25 days retention time. Thereby confirming the said invention.

### Illustration 2:

### 2) Dry paddy straw is used as substrate:

2.5 Kilograms of Paddy straw with total solids (TS) of about 88 to 90 percent is allowed to undergo the said biomethanation process. The following results are observed:

| Sr. No. | Substrate | Digester to Gas Ratio | Biogas Potential recovery (Liters/kg T.S.) | Retention Time (Days) | Expected Biogas production (liters/day) | Actual Biogas production (liters/day) |
|---|---|---|---|---|---|---|
| I | Paddy Straw(dry) | 1.1 - 1.2 | 260 - 300 | 18 | 700 | 600 - 700 |

### Observations:

The conventional wet type biogas digester producing biogas at similar gas potential recovery required over 25 days retention time, the route followed by the said process takes 18 days retention time to produce biogas. Thus confirming the said process.

### ADVANTAGES OF THE PRESENT INVENTION:

The process reduces the overall hydraulic retention time required for the biomethanation process, thereby reducing the size of the biogas digester of equivalent capacity.

The process describes a solid state anaerobic fermentation, thus the invention overcomes the problem of scum formation, thereby increasing the efficiency of the biomethanation process.

The process allows use of mixed and / or multiple solid feeds as substrates for anaerobic digestion to produce biogas and manures.

The process describes dual biomethanation, wherein leachate from the solid state methane digester is utilized in liquid state methane digester to maximize biogas production.

The process produces less waste water than wet digestion systems and therefore requires less equipment for managing this effluent stream.

The process involves minimum auxiliary power / energy consumption.

## Claims

1. An apparatus for biomethanation of biomass by combined solid and liquid state anaerobic fermentation, said apparatus comprising:
two or more different anaerobic digesters (2, 5), comprising at least a solid state digester (2) and a liquid state digester (5), the two or more different digesters (2, 5) for generating biogas in solid and liquid (dual) states using mixed solid feeds as substrates;
a culture preparation tank / unit (10) for preparing feed from non-digested feed material for said process;
a filtration unit / percolation unit (11) for draining excess water from the non-digested feed material; and
a manure preparation unit (12) for preparing manure from the non-digested feed material;
wherein
the solid state digester (2) has an outlet/conduit (18) in communication with the culture preparation tank/unit (10),
wherein solid non digested feed material is collected in said culture preparation unit (10) to produce culture for the biomethanation process, and non-digested feed material from the culture preparation unit (10) is introduced into the solid state methane digester (2) with fresh biomass and is treated for manure preparation.

2. An apparatus as claimed in Claim 1, wherein the solid state methane digester (2) comprises:
a gas collecting fixed dome or other device to gather biogas generated from the reaction mixture;
a reaction chamber (3) for biomethanation process;
a vertical perforated unit (tube) (4) at the internal base of solid state digester (2) to facilitate a spray recirculation unit (13) or other arrangement for circulation connected to the other or liquid state methane digester (5) to facilitate percolation of leachates from the feed into the digester and maintaining the required temperature for the microbial activity for biogas generation;
control valves (15) for controlling flow of biogas from both the digesters (2, 5) into a common biogas collecting unit (7); and
a solid / slurry handling pump (14) to control the flow of leachates from the liquid state methane digester (5) to the solid state methane digester (2),
wherein
the number of said solid state methane digester (2) varies depending on the retention time of the biomass used for biogas generation.

3. An apparatus as claimed in Claim 1, wherein the leachates produced from the solid state methane digester (2) are utilized to generate biogas into another or liquid state methane digester (5) with addition of desired microbes.

4. An apparatus as claimed in Claim 3, wherein the leachates produced are recirculated through the anaerobic digesters to enhance microbial degradation of substrates and increase biogas production.

5. An apparatus as claimed in Claim 1, wherein manure is produced in the manure preparation unit (12) through the step of filtration of non-digested material until the desired carbon to nitrogen ratio is obtained as is required for manure.

6. An apparatus as claimed in Claim 1, wherein mixed solid feeds are used as substrates for anaerobic digestion to produce biogas and manures.

7. An apparatus as claimed in Claim 6, wherein the total solids of mixed and/or multiple solid feeds used is in the range of 15 to 20 percent.

8. An apparatus as claimed in Claim 1, wherein the substrates used are agro residues, oilcakes like paddy straw, wheat straw, maize, Napier grass, press mud, castor, sal, food waste, biodegradable municipal waste and alike.

9. Biomethanation of biomass by combined solid and liquid state anaerobic fermentation comprising the use of an apparatus as claimed in any one of Claims 1 to 8.

## Patentansprüche

1. Vorrichtung zur Biomethanation von Biomasse durch kombinierte anaerobe Fest- und Flüssigstoff-Fermentation, wobei besagte Vorrichtung Folgendes umfasst:
zwei oder mehr verschiedene anaerobe Digestoren (2, 5), umfassend mindestens einen Feststoff-Digestor (2) und einen Flüssigstoff-Digestor (5), wobei die zwei oder mehr verschiedenen Digestoren (2, 5), zum Erzeugen von Biogas im festen und flüssigen (dualen) Zustand, gemischte Feststoffeinspeisungen als Substrate benutzen;
einen (eine) Kulturaufbereitungstank/-einheit (10) zum Aufbereiten von Einspeisungen aus nicht verdautem Einspeisungsmaterial für besagtes Verfahren;
eine Filtrationseinheit/Perkolationseinheit (11) zum Ablassen von überschüssigem Wasser aus dem nicht verdauten Einspeisungsmaterial; und
eine Mistaufbereitungseinheit (12) zum Aufbereiten von Mist aus dem nicht verdauten Einspeisungsmaterial;
worin
der Feststoff-Digestor (2) einen (eine) Auslass/Leitung (18), der (die) mit dem (der) Kulturaufbereitungstank/-einheit (10) in Verbindung steht, aufweist,
worin festes, nicht verdautes Einspeisungsmaterial in besagter Kulturaufbereitungseinheit (10) gesammelt wird, um eine Kultur für das Biomethanationsverfahren zu produzieren, und
nicht verdautes Einspeisungsmaterial aus der Kulturaufbereitungseinheit (10) in den Feststoff-Methan-Digestor (2) mit frischer Biomasse eingebracht wird und zur Mistaufbereitung behandelt wird.

2. Vorrichtung nach Anspruch 1, worin der Feststoff-Methan-Digestor (2) Folgendes umfasst:
eine Gas sammelnde feste Kuppel oder sonstige Einrichtung zum Zusammentragen von aus der Reaktionsmischung erzeugtem Biogas;
eine Reaktionskammer (3) für das Biomethanationsverfahren;
eine vertikale perforierte Einheit (Röhre) (4) an der internen Basis des Feststoff-Digestors (2), um eine Sprayrezirkulationseinheit (13) oder sonstige Anordnung zur Zirkulation in Verbindung mit dem anderen oder Flüssigstoff-Methan-Digestor (5) zu ermöglichen, um die Perkolation von Sickerwässern aus der Einspeisung in den Digestor und Aufrechterhalten der erforderlichen Temperatur für die mikrobielle Aktivität zur Biogaserzeugung zu ermöglichen;
Regelventile (15) zum Regeln der Strömung von Biogas aus beiden der Digestoren (2, 5) in eine gemeinsame Biogas-Sammeleinheit (7); und
eine Feststoff-/Jauche-Förderpumpe (14) zum Regeln der Strömung von Sickerwässern vom Flüssigstoff-Methan-Digestor (5) zum Feststoff-Methan-Digestor (2),
worin
die Anzahl der besagten Feststoff-Methan-Digestoren (2) je nach der Retentionszeit der zur Biogaserzeugung benutzten Biomasse schwankt.

3. Vorrichtung nach Anspruch 1, worin die vom Feststoff-Methan-Digestor (2) produzierten Sickerwässer benutzt werden, um Biogas in einem anderen oder Flüssigstoff-Methan-Digestor (5) mit Zugabe gewünschter Mikroben zu erzeugen.

4. Vorrichtung nach Anspruch 3, worin die produzierten Sickerwässer durch die anaeroben Digestoren hindurch rezirkuliert werden, um mikrobiellen Abbau von Substraten zu verbessern und Biogasproduktion zu steigern.

5. Vorrichtung nach Anspruch 1, worin Mist in der Mistaufbereitungseinheit (12) durch den Schritt der Filtration nicht verdauten Materials produziert wird, bis das gewünschte Kohlenstoff-Stickstoff-Verhältnis wie für Mist erforderlich erzielt wird.

6. Vorrichtung nach Anspruch 1, worin gemischte Feststoffeinspeisungen als Substrate für anaerobe Digestion zum Produzieren von Biogas und Misten benutzt werden.

7. Vorrichtung nach Anspruch 6, worin die Gesamtfeststoffe von benutzten gemischten und/oder multiplen Feststoffeinspeisungen im Bereich von 15 bis 20 Prozent liegen.

8. Vorrichtung nach Anspruch 1, worin es sich bei den benutzten Substraten um landwirtschaftliche Rückstände, Ölkuchen wie Reisstroh, Weizenstroh, Mais, Elefantengras, Pressschlamm, Rizinus, Sal, Lebensmittelabfall, bioabbaubaren Stadtmüll und dergleichen handelt.

9. Biomethanation von Biomasse durch kombinierte anaerobe Fest- und Flüssigstoff-Fermentation umfassend die Verwendung einer Vorrichtung nach einem der Ansprüche 1 bis 8.

## Revendications

1. Appareil de biométhanisation de biomasse par fermentation anaérobie combinée aux états solide et liquide, ledit appareil comprenant :
au moins deux digesteurs anaérobies différents (2, 5), comprenant au moins un digesteur à l'état solide (2) et un digesteur à l'état liquide (5), les au moins deux digesteurs (2, 5) permettant de générer un biogaz aux états solide et liquide (deux états) au moyen d'alimentations de solides mixtes en guise de substrats ;
une cuve/unité de préparation de culture (10) permettant de préparer l'alimentation à partir de matière d'alimentation non digérée pour ledit processus ;
une unité de filtration/percolation (11) permettant de drainer l'eau en excès de la matière d'alimentation non digérée ; et
une unité de préparation d'engrais organique (12) permettant de préparer de l'engrais organique à partir de la matière d'alimentation non digérée ; le digesteur à l'état solide (2) comportant une sortie/conduite (18) en communication avec la cuve/unité de préparation de culture (10),
la matière d'alimentation non digérée solide étant collectée dans ladite unité de préparation de culture (10) afin de produire une culture pour le processus de biométhanisation, et
la matière d'alimentation non digérée en provenance de l'unité de préparation de culture (10) étant introduite dans le digesteur de méthane à l'état solide (2) avec de la biomasse fraîche et étant traité en vue de la préparation de l'engrais organique.

2. Appareil selon la revendication 1, dans lequel le digesteur de méthane à l'état solide (2) comprend :
un dôme fixe de collecte de gaz ou un autre dispositif permettant de recueillir du biogaz à partir du mélange réactionnel ;
une chambre de réaction (3) pour le processus de biométhanisation ;
une unité perforée verticale (tube) (4) au niveau de la base interne du digesteur à l'état solide (2) permettant à une unité de recirculation par pulvérisation (13) ou à un autre agencement de circulation connecté à un autre digesteur de méthane ou au digesteur de méthane à l'état liquide (5) de faciliter la percolation de lixiviats provenant de l'alimentation dans le digesteur et de maintenir la température requise pour l'activité microbienne pour la génération de biogaz ;
des vannes de régulation (15) permettant de réguler le débit de biogaz des deux digesteurs (2, 5) dans une unité de collecte de biogaz commune (7) ; et
une pompe de traitement de matières solides/boues (14) pour réguler le débit des lixiviats du digesteur de méthane à l'état liquide (5) au digesteur de méthane à l'état solide (2),
le nombre de digesteurs de méthane à l'état solide (2) variant selon la durée de retenue de la biomasse utilisée pour la génération de biogaz.

3. Appareil selon la revendication 1, dans lequel les lixiviats produits par le digesteur de méthane à l'état solide (2) sont utilisés pour générer du biogaz un autre digesteur de méthane ou un digesteur de méthane à l'état liquide (5) ajout des microbes souhaités.

4. Appareil selon la revendication 3, dans lequel les lixiviats produits sont recirculés dans les digesteurs anaérobies afin d'améliorer la dégradation microbienne des substrats et augmenter la production de biogaz.

5. Appareil selon la revendication 1, dans lequel de l'engrais organique est produit dans l'unité de préparation d'engrais organique (12) au moyen de l'étape de filtration de la matière non digérée jusqu'à ce que le rapport carbone-azote souhaité soit obtenu selon les besoins requis pour l'engrais organique.

6. Appareil selon la revendication 1, dans lequel des alimentations de solides mixtes sont utilisées en guise de substrats pour la digestion anaérobie afin de produire du biogaz et des engrais organiques.

7. Appareil selon la revendication 6, dans lequel les solides totaux des alimentations de solides mixtes et/ou multiples utilisées sont de l'ordre de 15 à 20 %.

8. Appareil selon la revendication 1, dans lequel les substrats utilisés sont des agro-résidus, des tourteaux tels que de la paille de riz, de la paille de blé, du maïs, de l'herbe de napier, des boues de pressage, du ricin, du sel, des déchets alimentaires, des déchets municipaux biodégradables et des substrats analogues.

9. Biomasse de biométhanisation par fermentation anaérobie combinée aux états solide et liquide, comprenant l'utilisation d'un appareil selon l'une quelconque des revendications 1 à 8.
